# EUROPEAN PATENT APPLICATION

(11) **EP 4 501 412 A1**
(43) Date of publication of application: **05.02.2025**
(21) Application number: 23780200.4
(22) Date of filing: 24.03.2023
(51) Int. Cl.: A61Q 1/00, A61Q 17/04, A61Q 19/00, A61K 8/365, A61K 8/37, A61K 8/92

(54) **OILY COMPOSITION AND COSMETIC PREPARATION**

(30) Priority: 28.03.2022 JP 2022051774
(71) Applicant: THE NISSHIN OILLIO GROUP, LTD., Tokyo 104-8285 (JP)
(72) Inventor: KACHI Hisanori, Yokohama-shi, Kanagawa 235-8558 (JP); OYAMA Keiichi, Yokohama-shi, Kanagawa 235-8558 (JP)
(74) Representative: Hasegawa, Kan
(86) International application number: PCT/JP2023/011886
(87) International publication number: WO 2023/190190

(57) **Abstract**

This oily composition comprises component (A), which is an ester having a solid fat content (SFC) at 25°C of 15-70% and a value obtained by dividing SFC at 35°C by SFC at 25°C, SFC (35°C)/SFC(25°C), of 0.08-0.60, component (B), which is an oil gellant having a solidification point of 50°C or higher, and component (C), which is an oily component that is liquid at 25°C, the amount of the component (B) being 0.05-10 parts by mass per 100 parts by mass of the component (A) and the amount of the component (C) being 10-500 parts by mass per 100 parts by mass of the component (A).

## Description

### [Technical Field]

The present invention relates to an oily composition and a cosmetic preparation.

Priority is claimed on Japanese Patent Application No. 2022-051774, filed in Japan on March 28, 2022, the content of which is incorporated herein by reference.

### [Background Art]

For cosmetics composed of oils, water, powders and the like, the texture including the spreading properties when applied to the skin and the compatibility with the skin is an important characteristic, and commercial products with various textures have been marketed in accordance with a concept associated with the particular cosmetic. Various components have been used to control those textures. For example, by incorporating a solid fat such as microcrystalline wax, a butter-like rich full-body sensation could be imparted when the product was placed on the skin and spread, thus producing a feeling of high quality. Further, by incorporating volatile components such as ethanol, and/or components such as menthol or peppermint oil which react with receptors associated with a sensation of cooling, a fresh cool sensation could be imparted during massage. Furthermore, paste-like oily components having a melting point near skin temperature can generate a cooling sensation due to the latent heat associated with melting, while also providing a feeling of compatibility as the product melts on the skin and changes state. Moreover, paste-like oils can impart a moist sensation to the skin following application, and are therefore useful components in improving the feeling associated with a product.

However, if a large amount of a solid fat is blended in order to achieve a rich full-body sensation immediately following application, there is a case in which a film-like feeling remains strongly during massage and even after use, and a sticky feeling is produced. In addition, components that are used to impart a fresh cool sensation during massage and after use, such as ethanol and menthol, can sometimes cause irritation depending on the person, and therefore care must be taken with the amounts used of these types of components. Further, although there are almost no cases where paste-like oily components having a melting point near skin temperature have caused irritation, the melting point is close to normal temperature, and therefore dissolution and precipitation tend to occur repeatedly during long-term storage, which causes a problem in which the solid gradually becomes more coarse and a rough grainy external appearance is imparted.

Patent Document 1 discloses a method for enhancing the cooling feeling of menthol in order to impart a fresh cool sensation to a cosmetic. However, there is a case in which the use of menthol is difficult, because there is a possibility that the use of menthol induces irritation.

### [Citation List]

### [Patent Document]

[Patent Document 1]
Japanese Unexamined Patent Application, First Publication No. Hei 11-209262

### [Summary of Invention]

### [Technical Problem]

In light of such a background, technology that would enable the production of a cosmetic that exhibits a rich full-body sensation immediately following application to the skin, yields a fresh feeling during use, produces no stickiness following use, imparts a moist sensation to the skin, has a smooth external appearance, and exhibits excellent stability with little change in external appearance even during storage would be very desirable.

The o present invention aims to provide a cosmetic preparation that exhibits a rich full-body sensation immediately following application to the skin, yields a fresh feeling during use, produces no stickiness following use, imparts a moist sensation to the skin, has a smooth glossy external appearance, and undergoes little change in external appearance even during storage, and also to provide an oily composition that is ideal to produce such a cosmetic preparation.

### [Solution to Problem]

Following intensive investigations, the inventors of the present invention discovered that a cosmetic preparation containing an oily composition prepared by mixing specific proportions of an oil or fat having solid fat contents (SFC) at 25°C and 35°C falling within specific ranges, an oily gelling agent, and an oily component that is liquid at 25°C, exhibits a rich full-body sensation immediately following application to the skin, yields a fresh feeling during use, produces no stickiness following use, imparts a moist sensation to the skin, has a smooth glossy external appearance, and undergoes little change in external appearance even during storage, thereby completing the present invention.

In other words, an oily composition and a cosmetic preparation according to the present invention are as described below.
[1] An oily composition containing:
   a component (A): an ester having a solid fat content (SFC) at 25°C within a range from 15 to 70% and having a value obtained by dividing the SFC at 35°C by the SFC at 25°C ([SFC (35°C)]/[SFC(25°C)]) that is within a range from 0.08 to 0.60;
   a component (B): an oily gelling agent having a solidification point of 50°C or higher; and
   a component (C): an oily component that is liquid at 25°C, wherein
   the amount of the component (B) relative to 100 parts by mass of the component (A) is within a range from 0.05 to 10 parts by mass, and the amount of the component (C) relative to 100 parts by mass of the component (A) is within a range from 10 to 500 parts by mass.
[2] The oily composition according to [1], wherein the ester of the component (A) is a glycerol derivative.
[3] The oily composition according to [1] or [2], wherein the ester of the component (A) is glyceryl tri(caprylate/caprate/myristate/stearate).
[4] The oily composition according to any one of [1] to [3], wherein the solidification point of the oily gelling agent of the component (B) is within a range from 60 to 90°C.
[5] The oily composition according to any one of [1] to [4], wherein the oily gelling agent of the component (B) is a glycerol derivative.
[6] The oily composition according to any one of [1] to [5], wherein the oily gelling agent of the component (B) is a material containing eicosanedioic acid within the structure thereof.
[7] The oily composition according to any one of [1] to [4], wherein the oily gelling agent of the component (B) is at least one selected from the group consisting of glyceryl (behenate/eicosanedioate), glyceryl tri(behenate/isostearate/eicosanedioate), polyglyceryl-10 (behenate/eicosanedioate), glyceryl tribehenate, 12-hydroxystearic acid, and candelilla wax.
[8] A cosmetic preparation containing the oily composition according to any one of [1] to [7].
[9] The cosmetic preparation according to [8], wherein the amount of the oily composition in the cosmetic preparation is within a range from 1 to 40% by mass relative to the total mass of the cosmetic preparation.
[10] A cosmetic preparation containing:
   a component (A): an ester having a solid fat content (SFC) at 25°C within a range from 15 to 70% and having a value obtained by dividing the SFC at 35°C by the SFC at 25°C ([SFC (35°C)]/[SFC(25°C)]) that is within a range from 0.08 to 0.60;
   a component (B): an oily gelling agent having a solidification point of 50°C or higher; and
   a component (C): an oily component that is liquid at 25°C, wherein
   the amount of the component (B) relative to 100 parts by mass of the component (A) is within a range from 0.05 to 10 parts by mass, and the amount of the component (C) relative to 100 parts by mass of the component (A) is within a range from 10 to 500 parts by mass.
[11] The cosmetic preparation according to [10], wherein the total amount of the component (A), the component (B) and the component (C) in the cosmetic preparation is within a range from 1 to 40% by mass relative to the total mass of the cosmetic preparation.
[12] The cosmetic preparation according to [10], containing the oily composition of any one of [1] to [7].
[13] The cosmetic preparation according to [12], wherein the amount of the oily composition in the cosmetic preparation is within a range from 1 to 40% by mass relative to the total mass of the cosmetic preparation.
[14] The cosmetic preparation according to any one of [10] to [13], further containing a component (D): water.
[15] The cosmetic preparation according to any one of [10] to [14], further containing a component (E): a surfactant.
[16] The cosmetic preparation according to any one of [10] to [15], further containing a component (F): a powder.

### [Advantageous Effects of Invention]

The oily composition and the cosmetic preparation according to the present invention exhibit a rich full-body sensation immediately following application to the skin, yield a fresh feeling during use, produce no stickiness following use, and impart a moist sensation to the skin.

Further, the oily composition and the cosmetic preparation according to the present invention have a smooth glossy external appearance, and undergo little change in external appearance even during storage.

### [Description of Embodiments]

### <Oily Composition>

The oily composition according to the present invention includes the component (A), the component (B) and the component (C) described below, wherein the amount of the component (B) relative to 100 parts by mass of the component (A) is within a range from 0.05 to 10 parts by mass, and the amount of the component (C) relative to 100 parts by mass of the component (A) is within a range from 10 to 500 parts by mass.
Component (A): an ester having a solid fat content (SFC) at 25°C within a range from 15 to 70% and having a value obtained by dividing the SFC at 35°C by the SFC at 25°C ([SFC (35°C)]/[SFC(25°C)]) that is within a range from 0.08 to 0.60,
Component (B): an oily gelling agent having a solidification point of 50°C or higher
Component (C): an oily component that is liquid at 25°C

First, the component (A) will be explained in detail.

An oil or fat having a SFC at 25°C within a range from 15 to 70% and having a value obtained by dividing the SFC at 35°C by the SFC at 25°C within a range from 0.08 to 0.60 is an oil or fat having specific values of the solid fat content as measured by the SFC measurement described below. The external appearance of the oil or fat at 25°C is in a paste-like state or a solid state. Hereinafter, the term "SFC at 35°C" may be abbreviated as "SFC (35°C)", the term "SFC at 25°C" may be abbreviated as "SFC (25°C)", and the phrase"value obtained by dividing the SFC at 35°C by the SFC at 25°C" may be abbreviated as "SFC (35°C/25°C)".

The SFC in an oil or fat is an index often used to show the temperature characteristics of margarine, chocolate or the like. One known method for measuring the SFC in an oil or fat involves placing a sample in a strong magnetic field to align spins of hydrogen atoms, irradiating the sample with electromagnetic waves of a specific wavelength to forcibly alter the spin alignment of the hydrogen atoms, and then evaluating the length of time taken for the spins of the hydrogen atoms to return to their original state. This measurement method utilizes the characteristic that spins of hydrogen atoms in a substance in a solid state return quickly, whereas spins of hydrogen atoms in a substance in a liquid state return slowly.

The SFC may be measured in accordance with the method disclosed in "Provision 1-1996 Solid Fat Content NMR Method" of the "Standard Methods for the Analysis of Fats and Oils", and may be measured, for example, using a commercially available equipment such as a "miniSpec mq-20" or "miniSpec SQ-20" manufactured by Bruker Corporation.

The SFC (25°C) in the component (A) in the present invention is within a range from 15 to 70%, and is preferably from 20 to 40%, and more preferably from 20 to 35%. Further, the SFC (35°C/25°C) in the component (A) is within a range from 0.08 to 0.60, and is preferably from 0.15 to 0.5, and more preferably from 0.2 to 0.4. When the SFC in the component (A) falls within the above-mentioned range, the cosmetic preparation containing the oily composition exhibits a rich full-body sensation immediately following application to the skin, effectively produces a fresh feeling during use, produces no stickiness following use, and imparts a moist sensation.

The "fresh feeling" felt during application of the composition to the skin is a sensation in which there is very little feeling of warmth caused by friction between the hand and the skin during massage, but rather a slight cooling sensation, as the composition blends into the skin. It is surmised that the fresh feeling is caused by components that melt at skin temperature undergoing a change in state from solid to liquid, which is accompanied by a loss of heat from the surrounding air.

Examples of the ester used favorably as the component (A) include esters containing a linear saturated fatty acid or sterol skeleton in the structure thereof. Specific examples of a component available as the component (A) include: glycerol derivatives having a glycerol skeleton in the structure thereof, such as natural oils or fats such as glyceryl tri(caprylate/caprate/myristate/stearate), hydrogenated coco-glycerides, bis-diglyceryl polyacyladipate-2, shea butter, hydrogenated palm oil, and hydrogenated rapeseed oil, and oils or fats obtained by partial hydrogenation of the natural oils or fats; and oils or fats obtained by ester synthesis such as dipentaerythrityl hexa(hydroxystearate/stearate/rosinate), dipentaerythrityl tetra(hydroxystearate/isostearate), dipentaerythrityl hexahydroxystearate, phytosteryl macadamia nut fatty acid ester, phytosteryl oleate, cholesteryl 12-hydroxystearate, bis(behenyl/isostearyl/phytosteryl) dimer dilinoleyl dimer dilinoleate, (phytosteryl/isostearyl/cetyl/stearyl/behenyl) dimer dilinoleate, and phytosteryl macadamia nut fatty acid ester. The ester of the component (A) included in the oily composition according to the present invention may be composed of a single ester or two or more esters.

Among them, at least one selected from the group consisting of glycerol derivatives is preferred, at least one selected from the group consisting of glyceryl tri(caprylate/caprate/myristate/stearate), bis-diglyceryl polyacyladipate-2, partially hydrogenated oils or fats (hydrogenated palm oil, hydrogenated rapeseed oil), dipentaerythrityl hexa(hydroxystearate/stearate/rosinate), dipentaerythrityl hexahydroxystearate, bis(behenyl/isostearyl/phytosteryl) dimer dilinoleyl dimer dilinoleate, (phytosteryl/isostearyl/cetyl/stearyl/behenyl) dimer dilinoleate, phytosteryl macadamia nut fatty acid ester and phytosteryl oleate is preferred, at least one selected from the group consisting of glyceryl tri(caprylate/caprate/myristate/stearate) and partially hydrogenated oils or fats is more preferred, and glyceryl tri(caprylate/caprate/myristate/stearate) is even more preferred.

Commercially available products may be used as the ester of the component (A). Examples of the commercially available products that may be used favorably include SALACOS 334, NOMCORT PHS, SALACOS HS, SALACOS PO(T), COSMOL 168ARV and COSMOL 168EV (all manufactured by The Nisshin OilliO Group, Ltd.), and SOFTISAN 649 (manufactured by IOI Oleo GmbH).

Next, the component (B) will be explained in detail.

An oily gelling agent is a component that causes thickening or gelling of an oil fraction by precipitating in the oil when added to the oil fraction, dissolved under heat, and then left to cool. The oily gelling agent of the component (B) is preferably a material having a solidification point of 50°C or higher, more preferably a solidification point of 55 to 100°C, and even more preferably a solidification point of 60 to 90°C, the solidification point being measured solely. There are many different types of oily gelling agent, including those that produce fibrous precipitation in the oil, and those that precipitate as very fine crystals, and any of these types may be used, provided the solidification point is 50°C or higher. When the solidification point is 50°C or higher, changes in the external appearance during storage can be effectively suppressed. When the solidification point of the oily gelling agent is not more than 100°C, the heating temperature required to allow dissolution during production of a cosmetic can be kept low, and handling also becomes easier, both of which are desirable.

Examples of the oily gelling agent available as the component (B) include: materials that precipitate in fibrous form in oils, thereby becoming intertwined to form a network structure, such as polyurethane, polysiloxane, dibutyl lauroyl glutamide, 12-hydroxystearic acid and dextrin fatty acid esters; and materials that precipitate as a multitude of fine crystals in oils, thereby forming a waxy gel, such as polyethylene wax, ceresin, candelilla wax, carnauba wax, glyceryl (behenate/eicosanedioate), polyglyceryl-10 (behenate/eicosanedioate), glyceryl tri(behenate/isostearate/eicosanedioate), glyceryl tribehenate and behenyl behenate. Among them, a material that forms a waxy gel is preferred, and a glycerol derivative is more preferred. Further, a material having a structure containing eicosanedioic acid is preferred. Specifically, at least one selected from the group consisting of glyceryl (behenate/eicosanedioate), glyceryl tri(behenate/isostearate/eicosanedioate), polyglyceryl-10 (behenate/eicosanedioate), glyceryl tribehenate, 12-hydroxystearic acid and candelilla wax is preferred, at least one selected from the group consisting of glyceryl (behenate/eicosanedioate), polyglyceryl (behenate/eicosanedioate), glyceryl tri(behenate/isostearate/eicosanedioate) and glyceryl tribehenate is more preferred, and glyceryl (behenate/eicosanedioate) is the most preferred. The oily gelling agent of the component (B) included in the oily composition according to the present invention may be composed of a single material or two or more materials.

The method for measuring the solidification point of the gelling agent of the component (B) may be any conventionally used method, and there are no particular limitations. For example, a method using differential scanning calorimetry (DSC) enables measurement to be conducted simply and with good reproducibility. When DSC is used, the solidification point can be measured by heating a sample to a temperature at which the gelling agent melts completely, and then cooling the sample at a constant rate to detect the temperature at which heat generation occurs.

Next, the component (C) will be explained in detail.

Examples of an oily component that is liquid at 25°C include: naturally derived oils such as soybean oil, olive oil and squalene; mineral oils such as liquid paraffin; mineral-derived synthetic oils such as hydrogenated polybutene and hydrogenated polydecene; and synthetic ester oils such as glyceryl tri(caprylate/caprate), polyglyceryl-2 tetraisostearate, cetyl ethylhexanoate and isopropyl palmitate. When the component exhibits fluidity at 25°C as per the description, any component may be used without any particular restrictions.

Specific examples of the oily component that is liquid at 25°C include: hydrocarbons such as mineral oil (may also be referred to as liquid paraffin), heavy liquid isoparaffin, α-olefin oligomers, squalane, polyisobutylene, polybutene and hydrogenated polyisobutylene; oils or fats such as olive oil, castor oil, jojoba oil, mink oil and macadamia nut oil; esters such as cetyl 2-ethylhexanoate, isopropyl myristate, isopropyl palmitate, octyldodecyl myristate, tridecyl isononanoate, glyceryl tri(caprylate/caprate), polyglyceryl diisostearate, glyceryl triisostearate (triisostearin), polyglyceryl triisostearate, diglyceryl triisostearate, polyglyceryl tetraisostearate, diglyceryl tetraisostearate, trioctanoin, diisostearyl malate, neopentyl glycol dioctanoate, propylene glycol didecanoate, isopropyl myristate and coconut alkyl (caprylate/caprate); and higher alcohols such as isostearyl alcohol, octyldodecanol and isohexadecyl alcohol. Further, ethylhexyl methoxycinnamate, which is generally used as an ultraviolet absorber, is also liquid at 25°C and may be used as the component (C). Among them, mineral oil (may also be referred to as liquid paraffin), squalane, hydrogenated polyisobutylene, tridecyl isononanoate, glyceryl tri(caprylate/caprate), glyceryl triisostearate, diglyceryl tetraisostearate, coconut alkyl (caprylate/caprate), and ethylhexyl methoxycinnamate and the like may be used favorably.

One of these oily components may be used alone, or a combination of at least two thereof may be used.

Next, the amounts of the component (A), the component (B) and the component (C) in the oily composition according to the present invention will be explained in detail.

The amount of the component (B) relative to 100 parts by mass of the component (A) is within a range from 0.05 to 10 parts by mass, preferably from 0.1 to 10 parts by mass, more preferably from 1 to 5 parts by mass, and even more preferably from 1 to 2.5 parts by mass. When the amount falls within the above-mentioned range, a cosmetic preparation containing the oily composition has a smooth and glossy external appearance, and undergoes little change in external appearance upon storage.

Further, the amount of the component (C) relative to 100 parts by mass of the component (A) is within a range from 10 to 500 parts by mass, preferably from 10 to 400 parts by mass, more preferably from 20 to 300 parts by mass, and even more preferably from 100 to 300 parts by mass. When the amount falls within the above-mentioned range, a cosmetic preparation containing the oily composition exhibits a rich full-body sensation immediately following application to the skin, effectively produces a fresh feeling during use, and produces a refreshing sensation following use.

The oily composition according to the present invention can be produced by mixing the component (A), the component (B) and the component (C). In such cases, the amounts of the component (A), the component (B) and the component (C) are adjusted to fall within the above-mentioned range.

The temperature during mixing is set to a temperature at which the component (B) is melted, and for example, is preferably 50°C to 75°C, more preferably 60°C to 75°C, and even more preferably 65°C to 75°C. Further, the mixing may be conducted by manual stirring using a spatula or the like, or by using a stirring device such as a homo mixer or a homo disper.

### <Cosmetic Preparation>

Next, a cosmetic preparation according to the present invention will be explained.

The cosmetic preparation according to the present invention contains the component (A), the component (B) and the component (C), wherein the amount of the component (B) relative to 100 parts by mass of the component (A) is within a range from 0.05 to 10 parts by mass, and the amount of the component (C) relative to 100 parts by mass of the component (A) is within a range from 10 to 500 parts by mass.

The component (A), the component (B) and the component (C) in the cosmetic preparation of the present invention may each be added separately to produce the cosmetic preparation. Alternatively, the cosmetic preparation may be prepared by blending the oily composition according to the present invention, namely the oily composition containing the component (A), the component (B) and the component (C), in the cosmetic preparation.

Furthermore, the cosmetic preparation according to the present invention may be a cosmetic preparation consisting of the component (A), the component (B) and the component (C), namely the unmodified oily composition according to the present invention, or may contain one or more other components in addition to the component (A), the component (B) and the component (C).

In those cases where other components are included, the total amount of the component (A), the component (B) and the component (C) relative to the total mass of the cosmetic preparation according to the present invention is preferably within a range from 1 to 40% by mass, more preferably from 5 to 30% by mass, and even more preferably from 10 to 25% by mass.

When the component (A), the component (B) and the component (C) are blended using the oily composition described above, the amount of the oily composition relative to the total mass of the cosmetic preparation according to the present invention is preferably within a range from 1 to 40% by mass, more preferably from 5 to 30% by mass, and even more preferably from 10 to 25% by mass.

Furthermore, the total amount of other components relative to the total mass of the cosmetic preparation according to the present invention is preferably within a range from 60 to 99% by mass, more preferably from 70 to 95% by mass, and even more preferably from 75 to 90% by mass.

Next, the components other than the component (A), the component (B) and the component (C) will be explained in detail.

Water may be added as a component (D) to the cosmetic preparation according to the present invention. Any component typically used in cosmetics may be used as the component (D) without any particular restrictions.

Although there are no particular limitations on the amount added of the component (D), it is preferable, for example that the amount be within a range from 5 to 90% by mass relative to the total mass of the cosmetic preparation.

A surfactant may be added as a component (E) to the cosmetic preparation according to the present invention. Any surfactant component typically used in cosmetics may be used as the component (E) without any particular restrictions, and examples thereof include: ionic surfactants such as fatty acid soaps and sodium lauroyl glutamate; nonionic surfactants such as polyglyceryl fatty acid esters such as polyglyceryl-3 polyricinoleate and polyoxyethylene derivatives such as sorbitan sesquioleate and polysorbate 80 (polyoxyethylene sorbitan oleate); and silicone-based surfactants such as PEG-10 dimethicone, cetyl PEG/PPG-10/1 dimethicone, and polyether-modified silicones. Among them, polyglyceryl-3 polyricinoleate, sorbitan sesquioleate, polysorbate 80, cetyl PEG/PPG-10/1 dimethicone, and polyether-modified silicones may be used particularly favorably.

There are no particular limitations on the amount added of the component (E), but it is preferable, for example, that the amount be within a range from 0.01 to 10% by mass relative to the total mass of the cosmetic preparation is preferred.

One of these surfactants may be used alone, or a combination of at least two thereof may be used.

A powder may be added as a component (F) to the cosmetic preparation according to the present invention. As the component (F), various powders having different particle sizes, color or material quality may be blended depending on purposes such as altering the skin appearance, controlling ultraviolet light, or imparting a dry slippery sensation. The powder included in the cosmetic preparation according to the present invention may be composed of a single powder or two or more powders.

Any powder component typically used in cosmetics may be used as the component (F) without any particular restrictions, and examples thereof include extender pigments, colored pigments, and pearl pigments.

Examples of the extender pigments include inorganic pigments such as silicic acid, silicic anhydride, magnesium silicate, talc, sericite, mica, kaolin, clay, bentonite, bismuth oxychloride, zirconium oxide, magnesium oxide, zinc oxide, aluminum oxide, calcium sulfate, barium sulfate, magnesium sulfate, calcium carbonate, and magnesium carbonate, and composite powders thereof; organic powders composed of polyamide, polyester, polypropylene, polystyrene, polyurethane, nylon, silicone resins, vinyl resins, urea resins, phenol resins, silicon resins, acrylic resins, melamine resins, epoxy resins, polycarbonate resins, divinylbenzene-styrene copolymers, silk powder, cellulose, Nε-lauroyl-L-lysine, metal salts of long-chain alkyl phosphates, N-mono-long chain alkyl acyl basic amino acids, and metal soaps, and composite powders thereof; and composite powders of the above-mentioned inorganic powders and the above-mentioned organic powders. The particle shape of these powders may be any shape, including spherical, plate-like, needle-like, granular or amorphous.

Examples of the colored pigments include: metal oxides such as titanium oxide, zinc oxide, yellow iron oxide, colcothar, black iron oxide, Prussian blue, ultramarine, chromium oxide and chromium hydroxide; metal complexes such as manganese violet and cobalt titanate; inorganic pigments such as carbon black; organic pigments such as tar-based dyes and lake pigments; and natural pigments such as carmine.

Examples of pearl pigments that may be used include pearl pigments composed of mica or synthetic phologopite coated with a colorant such as titanium oxide, iron oxide, silicon oxide, Prussian blue, chromium oxide, carmine, or an organic pigment. These powders may also be subjected to any of various surface treatments such as a water repellency treatment or a water and oil repellency treatment using typical techniques.

In the present invention, there are no particular limitations on the addition of these powders typically used in cosmetic preparations, and the powders may be added depending on the purpose of the cosmetic preparation. Although an appropriate amount of such powders in the cosmetic preparation differs depending on the type of he cosmetic preparation, the amount of powders relative to the total mass of the cosmetic preparation is preferably 70 to 98% by mass in the case of a powder foundation, rouge or eye shadow, is preferably 5 to 30% by mass in the case of a water-in-oil emulsion foundation, emulsion-type eye shadow or sunscreen, and is preferably 2 to 15% by mass in the case of a lipstick.

The cosmetic preparation according to the present invention may also include an additive typically added to cosmetic preparations within a range in which effects achieved of the present invention are not impaired.

Examples of the additive include oily components (excluding the component (A), the component (B) and the component (C)), antioxidants, auxiliary antioxidants, preservatives, ultraviolet absorbers, monohydric alcohols, polyhydric alcohols, water-soluble polymers, pH regulators, inorganic salts, salts of organic acids, chelating agents, vitamins, organic solvents, fragrances, various extracts, and ultraviolet scattering agents. One of these additives may be used alone, or a combination of at least two thereof may be used.

The oily component is an oily component other than the component (A), the component (B) and the component (C), and examples thereof include fatty acids, higher alcohols, silicone oils and silicone resins. Specific examples thereof include lauric acid, myristic acid, palmitic acid, stearic acid, cetanol, stearyl alcohol, behenyl alcohol, dimethicone, phenylmethicone, and (dimethicone/(PEG-10/15)) crosspolymer.

Examples of the antioxidants include oil-soluble vitamin C derivatives, tocopherols, derivatives and salts thereof, dibutylhydroxytoluene, butylhydroxyanisole, and gallic acid esters. One of the antioxidants may be used alone, or a combination of at least two thereof may be used.

Examples of the auxiliary antioxidants include phosphoric acid, citric acid, maleic acid, malonic acid, succinic acid, fumaric acid, cephalin, hexametaphosphate, phytic acid, and ethylenediaminetetraacetic acid. One of the auxiliary antioxidants may be used alone, or a combination of at least two thereof may be used.

Examples of the preservatives include methylparaben, ethylparaben, butylparaben, and phenoxyethanol. One of the preservatives may be used alone, or a combination of at least two thereof may be used.

Examples of the ultraviolet absorbers include: benzoic acid-based ultraviolet absorbers such as para-aminobenzoic acid (hereinafter, abbreviated as PABA), PABA monoglyceryl ester, N,N-dipropoxy PABA ethyl ester, N,N-diethoxy PABA ethyl ester, l N,N-dimethyl PABA ethyester, and N,N-dimethyl PABA butyl ester; anthranilic acid-based ultraviolet absorbers such as homomenthyl-N-acetyl anthranilate; salicylic acid-based ultraviolet absorbers such as amyl salicylate, menthyl salicylate, homomenthyl salicylate, octyl salicylate, phenyl salicylate, benzyl salicylate, and p-isopropanolphenyl salicylate; cinnamic acid-based ultraviolet absorbers such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate, and glyceryl mono-2-ethylhexanoyl-diparamethoxy cinnamate; benzophenone-based ultraviolet absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonic acid salts, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone; 3-(4'-methylbenzilidine)-d,l-camphor, 3-benzilidine-d,l-camphor, urocanic acid, ethyl urocanate, 2-phenyl-5-methylbenzoxazole, 2,2'-hydroxy-5-methylphenylbenzotriazole, 2-(2'-hydroxy-5'-t-octylphenyl)benzotriazole, 2-(2'-hydroxy-5'-methylphenyl)benzotriazole, dibenzaladine, dianisoylmethane, 4-methoxy-4'-t-butyldibenzoylmethane, 5-(3,3-dimethyl-2-norbornylidine)-3-pentan-2-one, 2,4,6-trianilino-p-(carbo-2'-ethylhexyl-1'-oxy)-1,3,5-triazine, 4-tert-butyl-4'-methoxybenzoylmethane, ethylaminohydroxybenzoyl hexyl benzoate, ethylhexyltriazine, t-butylmethoxydibenzoylmethane, bis-ethylhexyloxyphenol methoxyphenyl triazine, diethylhexyl butamidotriazone, and oxybenzone-3. One of the ultraviolet absorbers may be used alone, or a combination of at least two thereof may be used.

Examples of the monohydric alcohols include methanol, ethanol, propanol, isopropanol, isobutyl alcohol, and t-butyl alcohol.

Examples of the polyhydric alcohols include propylene glycol (1,2-propanediol), 1,3-propanediol, 1,3-butylene glycol (1,3-butanediol), pentylene glycol (1,2-pentanediol), neopentylene glycol (2,2-dimethyl-1,3-propanediol), isoprene glycol (3-methyl-1,3-butanediol), dipropylene glycol, glycerol, diglycerol, polyglycerol, polyethylene glycol, pentaerythritol, dipentaerythritol, sorbitol, and sorbitan. One of the polyhydric alcohols may be used alone, or a combination of at least two thereof may be used.

The water-soluble polymers may be natural water-soluble polymers, semi-synthetic water-soluble polymers, or synthetic water-soluble polymers. The water-soluble thickener included in the cosmetic preparation according to the present invention may be composed of a single material, or a combination of at least two materials.

The water-soluble polymers may be natural water-soluble polymers, semi-synthetic water-soluble polymers, or synthetic water-soluble polymers. The water-soluble thickener included in the cosmetic preparation according to the present invention may be composed of a single material, or a combination of at least two materials.

Examples of the natural water-soluble polymers include: plant-based polymers such as agar, glucomannan, gum arabic, tragacanth gum, galactan, guar gum, carob gum, gum karaya, carrageenan, pectin, quince seed (marmelo), algae colloid (brown algae extract) and starches (rice, corn, potato, or wheat); microorganism-based polymers such as xanthan gum, dextran, succinoglycan and pullulan; and animal-based polymers such as collagen, casein, albumin and gelatin.

Examples of the semi-synthetic water-soluble polymers include starch-based polymers such as carboxymethyl starch and methyl hydroxypropyl starch; cellulose-based polymers such as methyl cellulose, nitrocellulose, methyl hydroxypropyl cellulose, sodium cellulose sulfate, hydroxypropyl cellulose, carboxymethyl cellulose, sodium carboxymethyl cellulose, crystalline cellulose and cellulose powder; and alginate-based polymers such as sodium alginate and propylene glycol alginate.

Examples of the synthetic water-soluble polymers include vinyl-based polymers such as polyvinyl alcohol, poly(vinyl methyl ether), polyvinylpyrrolidone and carboxyvinyl polymer; polyoxyethylene-based polymers such as polyethylene glycol 20,000, 40,000 and 60,000; polyoxyethylene-polyoxypropylene copolymer-based polymers; acrylic-based polymers such as sodium polyacrylate, poly(ethyl acrylate) and polyacrylamide; polyethyleneimine and cationic polymers.

Examples of the pH regulators include edetic acid, disodium edetate, citric acid, sodium citrate, sodium hydroxide, potassium hydroxide and triethanolamine. One of the pH regulators may be used alone, or a combination of at least two thereof may be used.

Examples of the inorganic salts include sodium chloride, potassium chloride, magnesium chloride, sodium sulfate, potassium sulfate and magnesium sulfate. Examples of the salts of organic acids include citric acid, malic acid, tartaric acid, and salts thereof, ascorbic acid and salts thereof, and ascorbic acid derivatives and salts thereof.

Examples of the chelating agents include disodium edetate, edetic acid salts, and hydroxyethane diphosphonic acid. One of the chelating agents may be used alone, or a combination of at least two thereof may be used.

Examples of the vitamins include vitamin A, vitamin B1, vitamin B2, vitamin B6, vitamin E, vitamin K, and derivatives thereof, pantothenic acid and derivatives thereof, and biotin.

Examples of the extracts include plant extracts such as aloe vera, witch hazel, hamamelis, cucumber, lemon, lavender and rose.

There are no particular limitations on the external appearance, the form, and the fluidity of the cosmetic preparation according to the present invention, and the external appearance may be transparent, semi-transparent or emulsified (cloudy), while the form may any form, including liquid, cream, solid and the like.

Preferable examples of the cosmetic preparation according to the present invention include: makeup cosmetics such as powder foundations, water-in-oil emulsion foundations, sunscreens, eye shadows, rouges and lipsticks; and skincare cosmetics such as milky lotions, creams, serums, and skin lotions. The cosmetic preparation is particularly preferably a skincare cosmetic, sunscreen cosmetic or foundation that requires a refreshing sensation upon use.

These cosmetic preparations may be produced using conventional methods with the exception of adding the prescribed amounts of the component (A), the component (B) and the component (C), or the oily composition of the present invention.

### [Examples]

The present invention will be explained below in further detail with reference to specific examples. However, the present invention is in no way limited by the content of these examples.

### [Production Example 1] Method for Synthesizing Bis-diglyceryl Polyacyladipate-2

A two-liter four-necked flask fitted with a stirrer, a gas inlet line, a cooling tube and a thermometer was charged with 166 g (1 mol) of diglycerol, 7.2 g (0.05 mol) of caprylic acid, 68.8 g (0.4 mol) of capric acid, 113.6 g (0.4 mol) of isostearic acid, 284 g (1 mol) of stearic acid, and 45 g (0.15 mol) of 12-hydroxystearic acid. The mixture was heated to 200 to 220°C while nitrogen was blown into the four-necked flask, followed by allowing the reaction to proceed for five hours while removing the produced water. Subsequently, 73 g (0.5 mol) of adipic acid was added thereto and the reaction was continued. The acid value of the reaction solution was measured periodically, and when the acid value fell to 5 mgKOH/g or lower, the reaction was halted, thus obtaining bis-diglyceryl polyacyladipate-2. The bis-diglyceryl polyacyladipate-2 had a SFC (25°C) of 18.4% and a SFC (35°C/25°C) of 0.22.

### [Production Example 2] Method for Synthesizing Bis(behenyl/isostearyl/phytosteryl) Dimer Dilinoleyl Dimer Dilinoleate

A two-liter four-necked flask fitted with a stirrer, a gas inlet line, a cooling tube and a thermometer was charged with 672 g (1.2 mol) of dimer dilinoleic acid, 159.6 g (0.6 mol) of dimer dilinoleyl alcohol, 326 g (1 mol) of behenyl alcohol, 28.4 g (0.1 mol) of isostearyl alcohol, and 41.4 g (0.1 mol) of phytosterol. While nitrogen was blown into the four-necked flask, the mixture was heated to 200 to 220°C, and the acid value of the reaction solution was measured periodically while removing the produced water. When the acid value fell to 5 mgKOH/g or lower, the reaction was halted, thus obtaining bis(behenyl/isostearyl/phytosteryl) dimer dilinoleyl dimer dilinoleate. The bis(behenyl/isostearyl/phytosteryl) dimer dilinoleyl dimer dilinoleate had a SFC (25°C) of 20.2% and a SFC (35°C/25°C) of 0.10.

### [Production Example 3] Method for Synthesizing

### (Phytosteryl/isostearyl/cetyl/stearyl/behenyl) Dimer Dilinoleate

A two-liter four-necked flask fitted with a stirrer, a gas inlet line, a cooling tube and a thermometer was charged with 560 g (1 mol) of dimer dilinoleic acid, 248.4 g (0.6 mol) of phytosterol, 113.6 g (0.4 mol) of isostearyl alcohol, 96.8 g (0.4 mol) of cetyl alcohol, 81 g (0.3 mol) of stearyl alcohol, and 97.8 g (0.3 mol) of behenyl alcohol. While nitrogen was blown into the four-necked flask, the mixture was heated to 200 to 220°C, and the acid value of the reaction solution was measured periodically while removing the produced water. When the acid value fell to 5 mgKOH/g or lower the reaction was halted, thus obtaining (phytosteryl/isostearyl/cetyl/stearyl/behenyl) dimer dilinoleate. The (phytosteryl/isostearyl/cetyl/stearyl/behenyl) dimer dilinoleate had a SFC (25°C) of 22.6% and a SFC (35°C/25°C) of 0.15.

### [Production Example 4] Method for Synthesizing Phytosteryl Macadamia Nut Fatty Acid Ester

A two-liter four-necked flask fitted with a stirrer, a gas inlet line, a cooling tube and a thermometer was charged with 279 g (1 mol) of macadamia nut fatty acid (acid value: 201 mgKOH/g, average molecular weight: 279) and 414 g (1 mol) of phytosterol. While nitrogen was blown into the four-necked flask, the mixture was heated to 200 to 220°C, and the acid value of the reaction solution was measured periodically while removing the produced water. When the acid value fell to 5 mgKOH/g or lower, the reaction was halted, thus obtaining phytosteryl macadamia nut fatty acid ester. The phytosteryl macadamia nut fatty acid ester had a SFC (25°C) of 50.0% and a SFC (35°C/25°C) of 0.32.

### [Production Example 5] Method for Synthesizing Polyglyceryl-10

### (Behenate/eicosanedioate)

A one-liter four-necked flask fitted with a stirrer, a gas inlet line, a cooling tube and a thermometer was charged with 148 g (0.2 mol) of polyglycerol-10 (hydroxyl value: 900 mgKOH/g), 408 g (1.2 mol) of behenic acid, and 35.4 g (0.1 mol) of eicosanedioic acid. While nitrogen was blown into the four-necked flask, the mixture was heated to 200 to 220°C, and the acid value of the reaction solution was measured periodically while removing the produced water. When the acid value fell to 5 mgKOH/g or lower, the reaction was halted. The solidification point of the obtained reaction product was 64°C.

### [Production Example 6] Method for Synthesizing Glyceryl Tribehenate

A one-liter four-necked flask fitted with a stirrer, a gas inlet line, a cooling tube and a thermometer was charged with 46 g (0.5 mol) of glycerol and 510 g (1.5 mol) of behenic acid. The four-necked flask was heated to 220°C to allow the reaction to proceed while removing the produced water. The acid value of the reaction solution was measured periodically, and when the acid value fell to 5 mgKOH/g or lower, the reaction was halted. The solidification point of the obtained reaction product was 57°C.

### <SFC Measurement Method>

The SFC of each ester was measured using the method described below. This measurement method is the method prescribed in "Provision 1-1996 Solid Fat Content NMR Method" of the "Standard Methods for the Analysis of Fats or Oils".
Measurement apparatus: "miniSpec SQ-20" manufactured by Bruker Corporation
Sample preparation: each of the components used as the component (A) was heated to 90°C, poured into a sample tube of ø10 mm, cooled to room temperature, and then left to stand sequentially for 30 minutes at each of 80°C, 60°C, 0°C and 20°C to prepare each measurement sample, followed by measuring SFC thereof at 25°C and 35°C.

### <Method for Measuring Solidification Point>

The solidification point of each easter or oily composition was measured using the method described below.
Apparatus used: "DSC7000X" manufactured by Hitachi High-Tech Science Corporation
Sample preparation: 5 to 10 mg of each sample was placed in a simple sealed cell.
Measurement conditions: the sample was heled at 110°C for five minutes, and then the amount of heat was measured while cooling the sample to 20°C at a cooling rate of 5°C/minute while. The temperature when heat generation started was read from the chart as the solidification point (°C).

The SFC information of esters including the component (A) used in examples and comparative examples are shown in Table 1. In Table 1, Nos. A-1 to A-10 were used in examples, and Nos. A-11 and A-12 were used in comparative examples.

**[Table 1]**

| Table 1 Esters used, and SFC (%) and SFC (35°C/25°C) thereof | | | | |
|---|---|---|---|---|
| No. | | SFC (25°C) | SFC (35°C) | SFC (35°C/25°C) |
| A-1 | Glyceryl tri(caprylate/caprate/myristate/stearate) | 24.4 | 8.0 | 0.33 |
| A-2 | Hydrogenated palm oil (iodine value: 40) | 64.0 | 37.8 | 0.59 |
| A-3 | Hydrogenated rapeseed oil (iodine value: 73) | 18.4 | 3.1 | 0.17 |
| A-4 | Bis-diglyceryl polyacyladipate-2 | 18.4 | 4.1 | 0.22 |
| | (Production Example 1) | | | |
| A-5 | Bis(behenyl/isostearyl/phytosteryl) dimer dilinoleyl dimer dilinoleate | 20.2 | 2.0 | 0.10 |
| | (Production Example 2) | | | |
| A-6 | (Phytosteryl/isostearyl/cetyl/stearyl/ behenyl) dimer dilinoleate | 22.6 | 3.4 | 0.15 |
| | (Production Example 3) | | | |
| A-7 | Dipentaerythrityl | 15.3 | 4.3 | 0.28 |
| | hexa(hydroxystearate/stearate/rosinate) | | | |
| A-8 | Dipentaerythrityl hexahydroxystearate | 21.6 | 12.6 | 0.58 |
| A-9 | Phytosteryl macadamia nut fatty acid ester | 50.0 | 16.1 | 0.32 |
| | (Production Example 4) | | | |
| A-10 | Phytosteryl oleate | 33.6 | 3.9 | 0.12 |
| A-11 | Dipentaerythrityl | 23.2 | 14.3 | 0.62 |
| | tetra(hydroxystearate/isostearate) | | | |
| A-12 | Microcrystalline wax | 80.0 | 72.3 | 0.90 |
| A-1: product name "SALACOS 334" manufactured by The Nisshin OilliO Group, Ltd. | | | | |
| A-7: product name "COSMOL 168ARV" manufactured by The Nisshin OilliO Group, Ltd. | | | | |
| A-8: product name "COSMOL 168M" manufactured by The Nisshin OilliO Group, Ltd. | | | | |
| A-10: product name "SALACOS PO(T)" manufactured by The Nisshin OilliO Group, Ltd. | | | | |
| A-11: product name "COSMOL 168EV" manufactured by The Nisshin OilliO Group, Ltd. | | | | |
| A-12: product name "Multiwax W-445" manufactured by Sonneborn LLC | | | | |

The gelling agents used in examples as the component (B) are shown in Table 2.

**[Table 2]**

| Table 2 Components (B) used, and solidification points thereof | | |
|---|---|---|
| No. | Component (B) | Solidification point (°C) |
| B-1 | Glyceryl (behenate/eicosanedioate) | 63 |
| B-2 | Glyceryl tri(behenate/isostearate/eicosanedioate) | 58 |
| B-3 | Polyglyceryl-10 (behenate/eicosanedioate) (Production Example 5) | 64 |
| B-4 | Glyceryl tribehenate (Production Example 6) | 57 |
| B-5 | 12-hydroxystearic acid | 72 |
| B-6 | Candelilla wax | 71 |
| B-1: product name "NOMCORT HK-G" manufactured by The Nisshin OilliO Group, Ltd. | | |
| B-2: product name "NOMCORT SG" manufactured by The Nisshin OilliO Group, Ltd. | | |
| B-5: product name "12-Hydroxystearic Acid" manufactured by FUJIFILM Wako Pure Chemical Corporation | | |
| B-6: product name "Pure Candelilla Wax No. 1" manufactured by CERARICA NODA Co., Ltd. | | |

Each oily composition (oil balm) including components as shown in Tables 3 to 6 was prepared by conducting heating to 70°C and mixing with a propeller for five minutes (rotational speed: 100 rpm), and then cooling to 25°C. Each obtained oily composition was evaluated in terms of rich full-body sensation immediately following application to the skin, fresh feeling during massage, stickiness following application, moist sensation following application, smoothness of external appearance, and storage stability. The product "COSMOL 44V" manufactured by The Nisshin OilliO Group, Ltd., was used as the diglyceryl tetraisostearate.

The evaluation results are shown in Tables 3 to 6. The numerical values in cells detailing components in the tables indicate % by mass values. Further, the term "SFC (25°C)" in the tables means "the solid fat content (%) at 25°C". Furthermore, the term "(B)/(A)" in the tables indicates the amount (parts by mass) of the component (B) in each sample when the amount of the component (A) is deemed 100 parts by mass. Similarly, the term "(C)/(A)" indicates the amount (parts by mass) of the component (C) when the amount of the component (A) is deemed 100 parts by mass.

### <Methods for Evaluating Sensations>

0.1 g of each cosmetic preparation for evaluation was placed on the inside of the forearm and massaged for 30 seconds using the four aligned fingers excluding the thumb of the opposite hand, and the rich full-body feeling immediately following application, the fresh feeling during use, the stickiness following application, and the moist sensation following application were evaluated by ten monitors.

### (Evaluation Criteria of Rich Full-Body Feeling Immediately Following Application)

A: Seven or more of the ten monitors reported feeling a rich full-body sensation.
B: Five or six of the ten monitors reported feeling a rich full-body sensation.
C: Three or four of the ten monitors reported feeling a rich full-body sensation.
D: Two or fewer of the ten monitors reported feeling a rich full-body sensation.

### (Evaluation Criteria of Fresh Feeling during Use)

A: Seven or more of the ten monitors reported feeling a fresh feeling.
B: Five or six of the ten monitors reported feeling a fresh feeling.
C: Three or four of the ten monitors reported feeling a fresh feeling.
D: Two or fewer of the ten monitors reported feeling a fresh feeling.

### (Evaluation Criteria of Absence of stickiness Following Use)

A: Seven or more of the ten monitors reported little stickiness.
B: Five or six of the ten monitors reported little stickiness.
C: Three or four of the ten monitors reported little stickiness.
D: Two or fewer of the ten monitors reported little stickiness.

### (Evaluation Criteria of Moist Sensation Following Use)

A: Seven or more of the ten monitors reported a moist sensation.
B: Five or six of the ten monitors reported a moist sensation.
C: Three or four of the ten monitors reported a moist sensation.
D: Two or fewer of the ten monitors reported a moist sensation.

### <Method for Evaluating External Appearance>

0.1 g of each cosmetic preparation for evaluation was placed on the inside of the forearm, and the external appearance when lightly spread for about 10 cm using the fingers of the opposite hand was evaluated.

### (Evaluation Criteria of External Appearance)

A: Seven or more of the ten monitors reported a smooth and glossy finish when spread across the skin.
B: Five or six of the ten monitors reported a smooth and glossy finish when spread across the skin.
C: Three or four of the ten monitors reported a smooth and glossy finish when spread across the skin.
D: Two or fewer of the ten monitors reported a smooth and glossy finish when spread across the skin.

### <Method for Evaluating Storage Stability>

Each cosmetic preparation for evaluation was placed in a glass screw-top vial, and the vial was then sealed and stored for 30 days while the temperature was varied alternately between 20°C (12 hours) and 0°C (12 hours). At the same time, another sample was stored at 25°C for 30 days for comparison. Following storage, a small amount of each sample was placed on a metal plate and spread thinly, and the storage stability was evaluated by visual inspection of the external appearance.

### (Evaluation Criteria of Storage Stability)

A: compared with storage at 25°C, either no difference or very little difference was observed.
B: compared with storage at 25°C, the gloss was slightly less.
C: compared with storage at 25°C, the gloss was less, and the surface was slightly rough.
D: compared with storage at 25°C, the gloss was significantly less, and crystals had precipitated to form coarse solid particles.

### (Evaluation Criteria of Overall Score)

Each evaluation result in terms of rich full-body sensation immediately following application, fresh feeling during use, stickiness following application, moist sensation following application, external appearance and storage stability were converted to a score wherein A=3, B=2, C=1 and D=0, and the product of all scores was used as the overall score (overall evaluation).

Samples for which the overall score was 35 or higher were adjudged to be samples having commercial value.

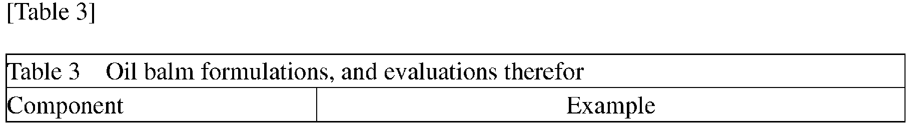

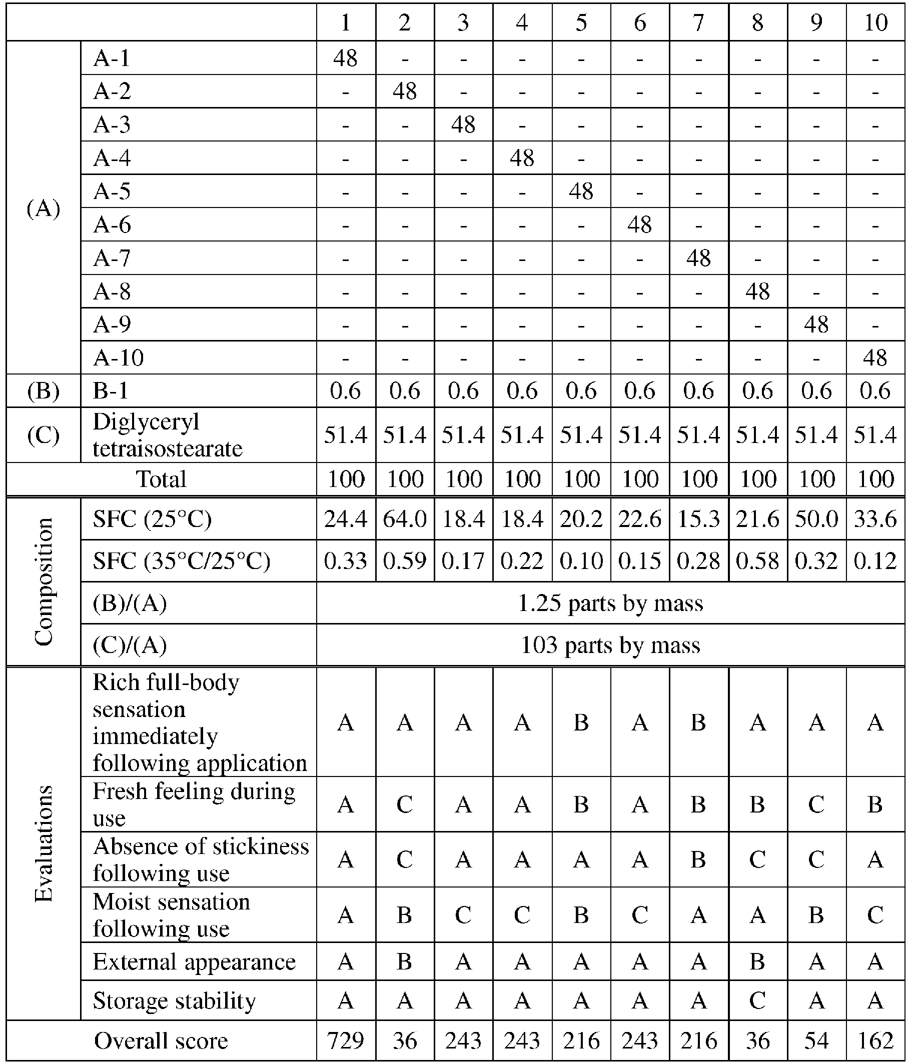

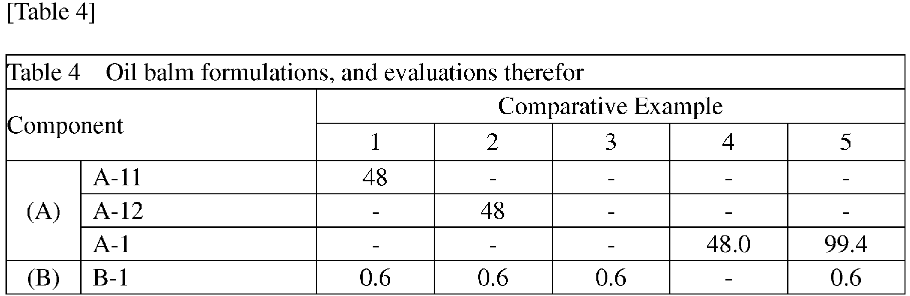

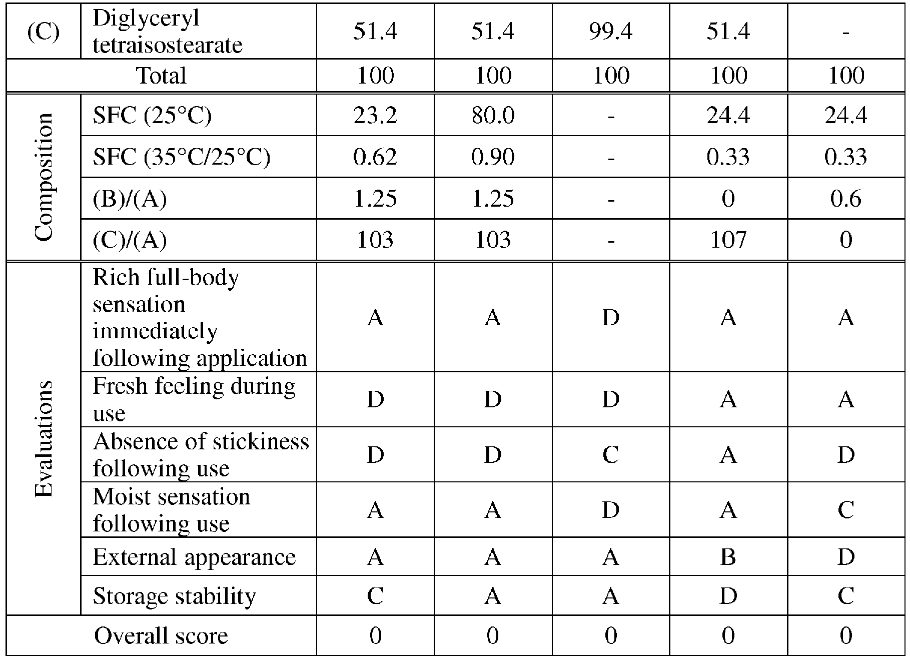

### [Table 5]

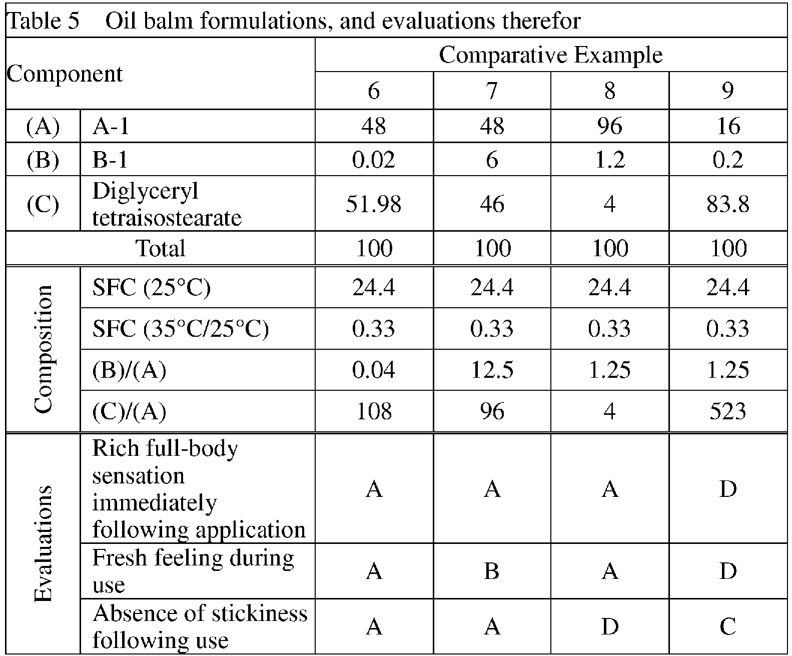

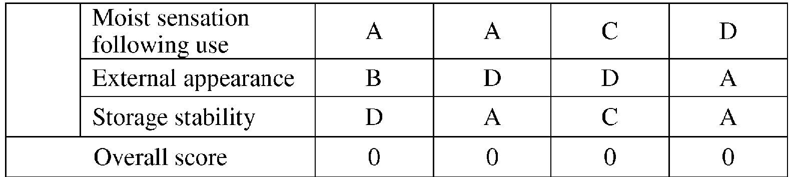

### [Table 6]

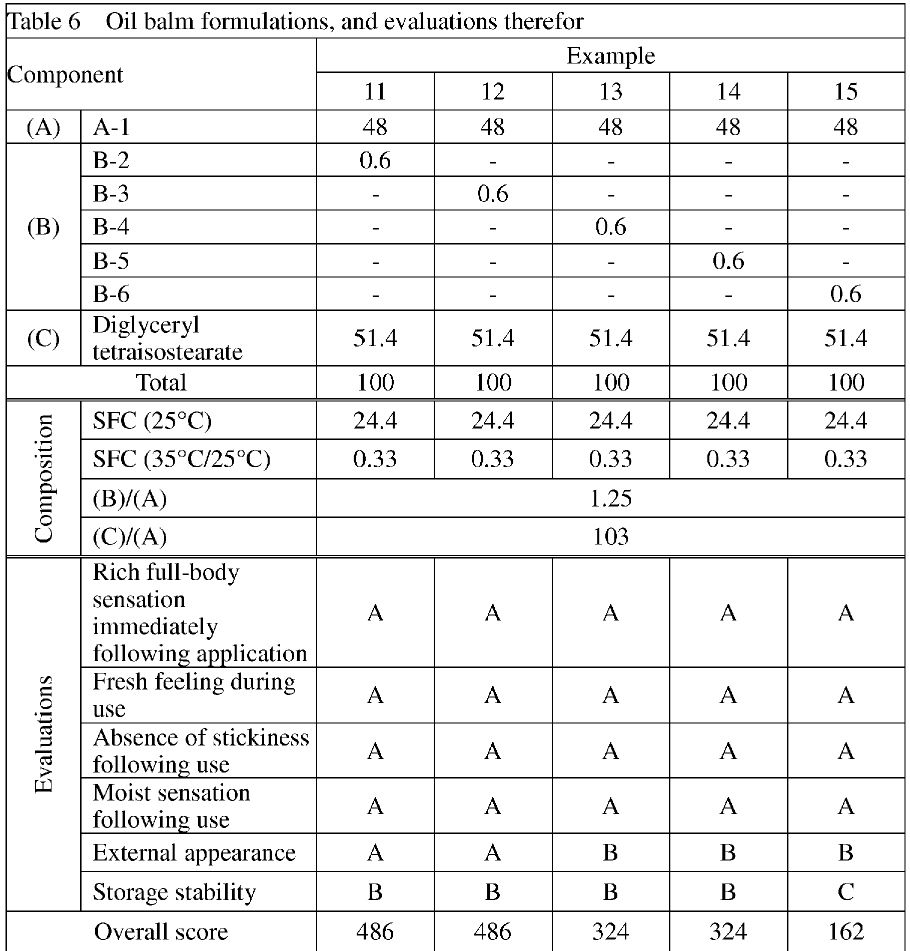

As shown in Tables 3 to 6, the oil balms of Examples 1 to 15 exhibited a rich full-body sensation immediately following application to the skin, produced a fresh feeling during use, yielded no stickiness following use, imparted a moist sensation to the skin, had a smooth external appearance, and exhibited excellent storage stability, with little change in external appearance even during storage. In contrast, the oil balms of Comparative Examples 1 and 2, in which the SFC (25°C/25°C) in the component (A) was larger than the range prescribed in the present invention, exhibited unsatisfactory results in terms of the fresh feeling during use and the absence of stickiness following use. Further, the oil balms of Comparative Examples 3 to 9 in which one of the components (A) to (C) was not contained or the blend ratio between the components was outside the ranges prescribed in the present invention. The oil balm of Comparative Example 3 in which the component (A) was not contained exhibited inferior results in terms of the rich full-body sensation immediately following application to the skin, the fresh feeling during use, and the moist sensation following use. The oil balm of Comparative Example 4 in which the component (B) was not contained had poor storage stability. The oil balm of Comparative Example 5 in which the component (C) was not contained exhibited stickiness following use, and exhibited inferior smoothness of the external appearance. The oil balm of Comparative Example 6, in which the amount added of the component (B) was small and the value of (B)/(A) was small, had poor storage stability. In contrast, the oil balm of Comparative Example 7, in which the amount added of the component (B) was large and the value of (B)/(A) was large, exhibited inferior smoothness of the external appearance. Moreover, the oil balm of Comparative Example 8, in which the amount added of the component (C) was small and the value of (C)/(A) was small, exhibited inferior smoothness of the external appearance. In contrast, the oil balm of Comparative Example 9, in which the amount added of the component (C) was large and the value of (C)/(A) was large, exhibited inferior results in terms of the rich full-body sensation immediately following application and the fresh feeling during use.

### [Oil-in-Water Moisturizing Milky Lotions]

Each oil-in-water moisturizing milky lotion including components as shown in Table 7 was prepared, and evaluated in terms of rich full-body sensation immediately following application to the skin, fresh feeling during massage, stickiness following application, moist sensation following application, smoothness of external appearance, and storage stability. The product "COSMOL 44V" manufactured by The Nisshin OilliO Group, Ltd., was used as the diglyceryl tetraisostearate, the product "Carnation" manufactured by Sonneborn LLC was used as the liquid paraffin, the product "PARLEAM 18" manufactured by NOF CORPORATION was used as the hydrogenated polyisobutene, the product "O.D.O" manufactured by The Nisshin OilliO Group, Ltd., was used as the glyceryl tri(caprylate/caprate), the product "KF-96 100cs" manufactured by Shin-Etsu Chemical Co., Ltd., was used as the dimethicone, the product "KALCOL 6870" manufactured by Kao Corporation was used as the cetanol, the product "EMALEX ET-8020" manufactured by NIHON EMULSION Co., Ltd., was used as the polysorbate 80, the product "COSMOL 82" manufactured by The Nisshin OilliO Group, Ltd., was used as the sorbitan sesquioleate, and the product "PEMULEN TR-1" manufactured by The Lubrizol Corporation was used as the acrylates / C10-30 alkyl acrylate crosspolymer.

Each oil-in-water moisturizing milky lotion was produced using steps A to C described below.
A: Components 1 to 9 shown in Table 7 were dissolved under heat at 70°C and then mixed uniformly with a spatula.
B: Components 12 to 17 shown in Table 7 were heated to 70°C and mixed uniformly with a spatula.
C: The components 10 and 11 shown in Table 7 were added to the mixture obtained in the step B, the mixture obtained in the step A was then added, the resulting mixture was mixed and emulsified for five minutes using a homo mixer (rotational rate: 5,000 rpm), and the mixture was then cooled to obtain a thick oil-in-water moisturizing milky lotion.

**[Table 7]**

| Table 7 Oil-in-water moisturizing milky lotions | | | | | | | |
|---|---|---|---|---|---|---|---|
| | | | Ex. 16 | Ex. 17 | C. Ex. 10 | C. Ex. 11 | C. Ex. 12 |
| No. | Component (raw material) | | % by mass | % by mass | % by mass | % by mass | % by mass |
| 1 | (A) | A-1 | 4 | - | - | 4 | 4 |
| 2 | | A-6 | - | 4 | - | - | - |
| 3 | (B) | B-1 | 0.1 | 0.1 | 0.1 | - | 0.1 |
| 4 | (C) | Diglyceryl tetraisostearate | 3 | 3 | 3 | 3 | - |
| 5 | | Liquid paraffin | 5 | 5 | 5 | 5 | - |
| 6 | | Hydrogenated polyisobutene | 1 | 1 | 1 | 1 | - |
| 7 | | Glyceryl tri(caprylate/caprate) | 3 | 3 | 3 | 3 | - |
| 8 | - | Dimethicone | 0.5 | 0.5 | 0.5 | 0.5 | 0.5 |
| 9 | - | Cetanol | 1 | 1 | 1 | 1 | 1 |
| 10 | (E) | Polysorbate 80 | 0.6 | 0.6 | 0.6 | 0.6 | 0.6 |
| 11 | | Sorbitan sesquioleate | 0.4 | 0.4 | 0.4 | 0.4 | 0.4 |
| 12 | - | 1,3-butylene glycol | 10 | 10 | 10 | 10 | 10 |
| 13 | - | Glycerol | 2 | 2 | 2 | 2 | 2 |
| 14 | - | Phenoxyethanol | 0.5 | 0.5 | 0.5 | 0.5 | 0.5 |
| 15 | - | Acrylates / C10-30 alkyl acrylate crosspolymer | 0.2 | 0.2 | 0.2 | 0.2 | 0.2 |
| 16 | - | Potassium hydroxide | 0.04 | 0.04 | 0.04 | 0.04 | 0.04 |
| 17 | (D) | Ion-exchanged water | 68.66 | 68.66 | 72.66 | 68.76 | 80.66 |
| Total | | | 100 | 100 | 100 | 100 | 100 |
| Composition | | (B)/(A) | 2.5 | 2.5 | - | 0 | 2.5 |
| | | (C)/(A) | 300 | 300 | - | 300 | 0 |
| Evaluation | Rich full-body sensation immediately following application | | A | A | D | A | A |
| | Fresh feeling during use | | A | A | A | A | A |
| | Absence of stickiness following use | | A | A | A | A | A |
| | Moist sensation following use | | A | C | D | A | D |
| | External appearance | | A | A | A | C | D |
| | Storage stability | | A | A | A | D | C |
| Overall Evaluation | | | 729 | 243 | 0 | 0 | 0 |

| | | | | | | | |
|---|---|---|---|---|---|---|---|
| (Ex.: Example, C. Ex: Comparative Example) | | | | | | | |

As shown in Table 7, the oil-in-water moisturizing milky lotions of Examples 16 and 17, which contained the component (A), the component (B) and the component (C), essential in the oily composition according to the present invention, in appropriate ratios, exhibited a rich full-body sensation immediately following application to the skin, produced a fresh feeling during use, yielded no stickiness following use, imparted a moist sensation to the skin, had a smooth external appearance, and exhibited excellent storage stability, with little change in external appearance even during storage. In contrast, the oil-in-water moisturizing milky lotions of Comparative Examples 10 to 12, in which one of the component (A), the component (B) and the component (C), essential in the oily composition according to the present invention, was not contained, did not realize satisfactory at least one of a rich full-body sensation immediately following application to the skin, a fresh feeling during use, absence of stickiness following use, a moist sensation following use, a smooth external appearance, and storage stability.

### [Oil-in-Water Moisturizing Creams]

Each oil-in-water moisturizing cream including components as shown in Table 8 was prepared and evaluated in terms of rich full-body sensation immediately following application to the skin, fresh feeling during massage, stickiness following application, moist sensation following application, smoothness of external appearance, and storage stability. The product "COSMOL 44V" manufactured by The Nisshin OilliO Group, Ltd., was used as the diglyceryl tetraisostearate, the product "O.D.O" manufactured by The Nisshin OilliO Group, Ltd., was used as the glyceryl tri(caprylate/caprate), the product "KF-96 100cs" manufactured by Shin-Etsu Chemical Co., Ltd., was used as the dimethicone, the product "KALCOL 6870" manufactured by Kao Corporation was used as the cetanol, the product "EMALEX ET-8020" manufactured by NIHON EMULSION Co., Ltd., was used as the polysorbate 80, the product "COSMOL 82" manufactured by The Nisshin OilliO Group, Ltd., was used as the sorbitan sesquioleate, and the product "PEMULEN TR-1" manufactured by The Lubrizol Corporation was used as the acrylates / C10-30 alkyl acrylate crosspolymer.

Each oil-in-water moisturizing cream was produced using steps A to C described below.
A: Components 1 to 9 shown in Table 8 were dissolved under heat at 70°C and then mixed uniformly with a spatula.
B: Components 12 to 17 shown in Table 8 were heated to 70°C and mixed uniformly with a spatula.
C: The components 10 and 11 from Table 8 were added to the mixture obtained in the step B, the mixture obtained in the step A was then added, the resulting mixture was mixed and emulsified for five minutes using a homo mixer (rotational rate: 5,000 rpm), and the mixture was then cooled to obtain an oil-in-water moisturizing cream.

**[Table 8]**

| Table 8 Oil-in-water moisturizing creams | | | | | | | |
|---|---|---|---|---|---|---|---|
| | | | Ex. 18 | Ex. 19 | Ex. 20 | C. Ex. 13 | C. Ex. 14 |
| No. | Component (raw material) | | % by mass | % by mass | % by mass | % by mass | % by mass |
| 1 | (A) | A-1 | 10 | - | - | - | 10 |
| 2 | | A-2 | - | 10 | - | - | - |
| 3 | | A-3 | - | - | 10 | - | - |
| 4 | (B) | B-1 | 0.2 | 0.2 | 0.2 | 0.2 | - |
| 5 | (C) | Diglyceryl tetraisostearate | 8 | 8 | 8 | 8 | 8 |
| 6 | | Glyceryl tri(caprylate/caprate) | 4 | 4 | 4 | 4 | 4 |
| 7 | | Squalane | 2 | 2 | 2 | 2 | 2 |
| 8 | - | Dimethicone | 1 | 1 | 1 | 1 | 1 |
| 9 | - | Cetanol | 2 | 2 | 2 | 2 | 2 |
| 10 | (E) | Polysorbate 80 | 1.2 | 1.2 | 1.2 | 1.2 | 1.2 |
| 11 | | Sorbitan sesquioleate | 0.8 | 0.8 | 0.8 | 0.8 | 0.8 |
| 12 | - | 1,3-butylene glycol | 8 | 8 | 8 | 8 | 8 |
| 13 | - | Glycerol | 4 | 4 | 4 | 4 | 4 |
| 14 | - | Phenoxyethanol | 0.5 | 0.5 | 0.5 | 05 | 0.5 |
| 15 | - | Acrylates / C10-30 alkyl acrylate crosspolymer | 0.2 | 0.2 | 0.2 | 0.2 | 0.2 |
| 16 | - | Potassium hydroxide | 0.04 | 0.04 | 0.04 | 0.04 | 0.04 |
| 17 | (D) | Ion-exchanged water | 58.06 | 58.06 | 58.06 | 68.06 | 58.26 |
| Total | | | 100 | 100 | 100 | 100 | 100 |
| Composition | | (B)/(A) | 2 | 2 | 2 | - | 0 |
| | | (C)/(A) | 140 | 140 | 140 | - | 140 |
| Evaluation | | Rich full-body sensation immediately following application | A | A | A | D | A |
| | | Fresh feeling during use | A | A | A | A | A |
| | | Absence of stickiness following use | A | B | A | A | A |
| | | Moist sensation following use | A | A | B | D | A |
| | | External appearance | A | A | A | A | C |
| | | Storage stability | A | A | A | A | D |
| Overall Evaluation | | | 729 | 486 | 486 | 0 | 0 |

| | | | | | | | |
|---|---|---|---|---|---|---|---|
| (Ex.: Example, C. Ex.: Comparative Example) | | | | | | | |

As shown in Table 8, the oil-in-water moisturizing creams of Examples 18 to 20, which contained the component (A), the component (B) and the component (C), essential in the oily composition according to the present invention, in appropriate ratios, exhibited a rich full-body sensation immediately following application to the skin, produced a fresh feeling during use, yielded no stickiness following use, imparted a moist sensation to the skin, had a smooth external appearance, and exhibited excellent storage stability, with little change in external appearance even during storage. In contrast, the oil-in-water moisturizing milky lotions of Comparative Examples 13 and 14, in which either the component (A) or the component (B), essential in the oily composition according to the present invention, was not contained, did not realize satisfactory at least one of a rich full-body sensation immediately following application to the skin, a fresh feeling during use, absence of stickiness following use, a moist sensation following use, a smooth external appearance, and storage stability.

### [Water-in-Oil Emulsion Foundations]

Each water-in-oil emulsion foundation including components as shown in Table 9 was prepared and evaluated in terms of rich full-body sensation immediately following application to the skin, fresh feeling during massage, stickiness following application, moist sensation following application, smoothness of external appearance, and storage stability. The product "KF-96 10cs" manufactured by Shin-Etsu Chemical Co., Ltd., was used as the dimethicone, the product "KSG-16" manufactured by Shin-Etsu Chemical Co., Ltd., was used as the mixture of (dimethicone / vinyl dimethicone) crosspolymer and cyclopentasiloxane, the product "KSP-100" manufactured by Shin-Etsu Chemical Co., Ltd., was used as the (vinyl dimethicone / methicone silsesquioxane) crosspolymer, the product "KF-6017" manufactured by Shin-Etsu Chemical Co., Ltd., was used as the polyether-modified silicone, the product "Soldoc PGPR" manufactured by IQL was used as the polyglyceryl-3 polyricinoleate, and the product "Bentone 38"

### manufactured by Elementis was used as the organic-modified clay mineral.

Each water-in-oil emulsion foundation was produced using steps A to C described below.
A: Components 1 to 13 shown in Table 9 were heated to 80°C, mixed for one minute using a homo mixer (rotational rate: 2,000 rpm) and subsequently cooled to 60°C, followed by adding components 14 to 23 shown in Table 9 thereto and then conducting mixing for five minutes using a homo mixer (rotational rate: 2,000 rpm).
B: Components 24 to 29 shown in Table 9 were heated to 60°C and mixed uniformly with a spatula.
C: The mixture obtained in the step B was added to the dispersion obtained in the step A, and the resulting mixture was mixed and emulsified for 5 minutes using a homo mixer (rotational rate: 5,000 rpm).
D: The emulsion obtained in the step C was cooled to 40°C, components 30 to 32 shown in Table 9 were added thereto, and then the resulting mixture was cooled to room temperature to obtain a liquid water-in-oil emulsion foundation.

**[Table 9]**

| Table 9 Water-in-oil emulsion foundations | | | | | | | |
|---|---|---|---|---|---|---|---|
| | | | Ex. 21 | Ex. 22 | Ex. 23 | C. Ex. 15 | C. Ex. 16 |
| No. | Component (raw material) | | % by mass | % by mass | % by mass | % by mass | % by mass |
| 1 | (A) | A-1 | 4 | - | - | - | 4 |
| 2 | | A-2 | - | 4 | - | - | - |
| 3 | | A-3 | - | - | 4 | - | - |
| 4 | (B) | B-1 | 0.05 | 0.05 | 0.05 | 0.05 | - |
| 5 | (C) | Triisostearin | 6 | 6 | 6 | 6 | 6 |
| 6 | | Coconut alkyl (caprylate/caprate) | 4 | 4 | 4 | 4 | 4 |
| 7 | | Mineral oil | 2 | 2 | 2 | 2 | 2 |
| 8 | - | Dimethicone | 4 | 4 | 4 | 4 | 4 |
| 9 | - | (Dimethicone / vinyl dimethicone) crosspolymer, cyclopentasiloxane | 1 | 1 | 1 | 1 | 1 |
| 10 | - | (Vinyl dimethicone / methicone silsesquioxane) crosspolymer | 1 | 1 | 1 | 1 | 1 |
| 11 12 | (E) | Polyether-modified silicone | 0.5 | 0.5 | 0.5 | 0.5 | 0.5 |
| | | Polyglyceryl-3 polvricinoleate | 0.4 | 0.4 | 0.4 | 0.4 | 0.4 |
| 13 | - | Organic-modified clay mineral | 0.5 | 0.5 | 0.5 | 0.5 | 0.5 |
| 14 | - | Decamethylcyclopenta siloxane | 16 | 16 | 16 | 16 | 16 |
| 15 | | Titanium oxide | 6 | 6 | 6 | 6 | 6 |
| 16 | | Zinc oxide | 2 | 2 | 2 | 2 | 2 |
| 17 | | Talc | 4 | 4 | 4 | 4 | 4 |
| 18 | | Mica | 2 | 2 | 2 | 2 | 2 |
| 19 | (F) | Red iron oxide | 0.2 | 0.2 | 0.2 | 0.2 | 0.2 |
| 20 | | Yellow iron oxide | 1.6 | 1.6 | 1.6 | 1.6 | 1.6 |
| 21 | | Black iron oxide | 0.2 | 0.2 | 0.2 | 0.2 | 0.2 |
| 22 | | Nylon | 2 | 2 | 2 | 2 | 2 |
| 23 | | Titanated mica | 2 | 2 | 2 | 2 | 2 |
| 24 | - | Ethanol | 5 | 5 | 5 | 5 | 5 |
| 25 | - | 1,3-butylene glycol | 4 | 4 | 4 | 4 | 4 |
| 26 | - | Glycerol | 1 | 1 | 1 | 1 | 1 |
| 27 | - | Phenoxyethanol | 0.5 | 0.5 | 0.5 | 0.5 | 0.5 |
| 28 | - | Sodium chloride | 0.5 | 0.5 | 0.5 | 0.5 | 0.5 |
| 29 | (D) | Ion-exchanged water | 29.39 | 29.39 | 29.39 | 29.39 | 29.39 |
| 30 | - | Antioxidant (dl-α-tocopherol) | 0.05 | 0.05 | 0.05 | 0.05 | 0.05 |
| 31 | - | Hyaluronic acid | 0.01 | 0.01 | 0.01 | 0.01 | 0.01 |
| 32 | - | Fragrance | 0.1 | 0.1 | 0.1 | 0.1 | 0.1 |
| Total | | | 100 | 100 | 100 | 100 | 100 |

| | | | | | | | |
|---|---|---|---|---|---|---|---|
| (Ex.: Example, C. Ex.: Comparative Example) | | | | | | | |

**[Table 10]**

| Table 10 Water-in-oil emulsion foundations | | | | | | |
|---|---|---|---|---|---|---|
| | | Ex. 21 | Ex. 22 | Ex. 23 | C. Ex. 15 | C. Ex. 16 |
| Composition | SFC (25°C) | 24.4 | 64.0 | 18.4 | - | 24.4 |
| | SFC (35°C/25°C) | 0.33 | 0.59 | 0.17 | - | 0.33 |
| | (B)/(A) | 2.5 | 2.5 | 2.5 | - | 0 |
| | (C)/(A) | 300 | 300 | 300 | - | 300 |
| Evaluation | Rich full-body sensation immediately following application | B | B | B | D | B |
| | Fresh feeling during use | A | A | A | A | A |
| | Absence of stickiness following use | A | B | A | A | A |
| | Moist sensation following use | A | A | B | D | A |
| | External appearance | A | A | A | A | C |
| | Storage stability | A | A | A | A | D |
| Overall Evaluation | | 486 | 324 | 324 | 0 | 0 |

| | | | | | | |
|---|---|---|---|---|---|---|
| (Ex.: Example, C. Ex.: Comparative Example) | | | | | | |

The evaluation results of each water-in-oil emulsion foundation are shown in Table 10. As shown in Table 10, the water-in-oil emulsion foundations of Examples 21 to 23, which contained the component (A), the component (B) and the component (C), essential in the oily composition according to the present invention, in appropriate ratios, exhibited a rich full-body sensation immediately following application to the skin, produced a fresh feeling during use, yielded no stickiness following use, imparted a moist sensation to the skin, had a smooth external appearance, and exhibited excellent storage stability, with little change in external appearance even during storage. In contrast, the oil-in-water moisturizing milky lotions of Comparative Examples 15 and 16, in which either the component (A) or the component (B), essential in the oily composition according to the present invention, was not contained, did not realize satisfactory at least one of a rich full-body sensation immediately following application to the skin, a fresh feeling during use, absence of stickiness following use, a moist sensation following use, a smooth external appearance, and storage stability.

### [Water-in-Oil Emulsion Sunscreens]

Each water-in-oil emulsion sunscreen including components as shown in Table 11 was prepared and evaluated in terms of rich full-body sensation immediately following application to the skin, fresh feeling during massage, stickiness following application, moist sensation following application, smoothness of external appearance, and storage stability. The product "KF-96 10cs" manufactured by Shin-Etsu Chemical Co., Ltd., was used as the dimethicone, the product "KF-7312J" manufactured by Shin-Etsu Chemical Co., Ltd., was used as the mixture of trimethoxysiloxysilicate and cyclopentasiloxane, the product "ABIL EM-90" manufactured by Evonik Industries AG was used as the cetyl dimethicone copolyol, the product "TIPAQU TTO-S2" manufactured by ISHIHARA SANGYO KAISHA, LTD., was used as the stearic acid-treated microparticulate titanium oxide, and a material obtained by treating the product "FINEX 25"manufactured by SAKAI CHEMICAL INDUSTRY CO., LTD., with 5% methylhydrogenpolysiloxane was used as the silicone-treated zinc oxide.

Each water-in-oil emulsion sunscreen was produced using steps A to D described below.
A: Components 1 to 16 were mixed uniformly with a spatula.
B: Components 17 to 22 were mixed uniformly with a spatula.
C: The mixture obtained in the step B was added to the mixture obtained in the step A, and the resulting mixture was mixed and emulsified for five minutes using a homo mixer (rotational rate: 5,000 rpm).
D: A resin bottle containing a stainless steel ball was filled with the liquid emulsion obtained in the step C.

**[Table 11]**

| Table 11 Water-in-oil emulsion sunscreens | | | | | | | |
|---|---|---|---|---|---|---|---|
| | | | Ex. 24 | Ex. 25 | Ex. 26 | C. Ex. 17 | C. Ex. 18 |
| No. | Component (raw material) | | % by mass | % by mass | % by mass | % by mass | % by mass |
| 1 | (A) | A-1 | 5 | - | - | - | 5 |
| 2 | | A-2 | - | 5 | - | - | - |
| 3 | | A-3 | - | - | 5 | - | - |
| 4 | (B) | B-1 | 0.05 | 0.05 | 0.05 | 0.05 | - |
| 5 | (C) | Glyceryl tri(caprylate/caprate) | 6 | 6 | 6 | 6 | 6 |
| 6 | | Isotridecyl isononanoate | 4 | 4 | 4 | 4 | 4 |
| 7 | | Ethylhexyl methoxycinnamate | 5 | 5 | 5 | 5 | 5 |
| 8 | - | Dimethicone | 5 | 5 | 5 | 5 | 5 |
| 9 | - | Cyclopentadimethicone | 12 | 12 | 12 | 12 | 12 |
| 10 | - | Trimethoxysiloxysilicate, cyclopentasiloxane | 2 | 2 | 2 | 2 | 2 |
| 11 | (E) | Cetyl dimethicone copolvol | 2 | 2 | 2 | 2 | 2 |
| 12 | | Sorbitan polyoxyethylene monooleate (20 mol) | 0.2 | 0.2 | 0.2 | 0.2 | 0.2 |
| 13 | | Sorbitan sesquioleate | 0.8 | 0.8 | 0.8 | 0.8 | 0.8 |
| 14 | (F) | Stearic acid-treated microparticulate titanium oxide | 5 | 5 | 5 | 5 | 5 |
| 15 | | Silicone-treated zinc oxide | 5 | 5 | 5 | 5 | 5 |
| 16 | | Nylon powder | 2 | 2 | 2 | 2 | 2 |
| 17 | - | 1,3-butylene glycol | 4 | 4 | 4 | 4 | 4 |
| 18 | - | Ethanol | 4 | 4 | 4 | 4 | 4 |
| 19 | - | Phenoxyethanol | 0.5 | 0.5 | 0.5 | 0.5 | 0.5 |
| 20 | - | Sodium chloride | 0.5 | 0.5 | 0.5 | 0.5 | 0.5 |
| 21 | (D) | Ion-exchanged water | 36.85 | 36.85 | 36.85 | 36.85 | 36.85 |
| 22 | - | Fragrance | 0.1 | 0.1 | 0.1 | 0.1 | 0.1 |
| Total | | | 100 | 100 | 100 | 100 | 100 |
| Composition | | (B)/(A) | 1 | 1 | 1 | - | 0 |
| | | (C)/(A) | 300 | 300 | 300 | - | 300 |
| Evaluation | | Rich full-body sensation immediately following application | B | B | B | D | B |
| | | Fresh feeling during use | A | A | A | A | A |
| | | Absence of stickiness following use | A | B | A | A | A |
| | | Moist sensation following use | A | A | B | D | A |
| | | External appearance | A | A | A | A | C |
| | | Storage stability | A | A | A | A | D |
| Overall Evaluation | | | 486 | 324 | 324 | 0 | 0 |

| | | | | | | | |
|---|---|---|---|---|---|---|---|
| (Ex.: Example, C. Ex.: Comparative Example) | | | | | | | |

As shown in Table 11, the water-in-oil emulsion foundations of Examples 24 to 26, which contained the component (A), the component (B) and the component (C), essential in the oily composition according to the present invention, in appropriate ratios, exhibited a rich full-body sensation immediately following application to the skin, produced a fresh feeling during use, yielded no stickiness following use, imparted a moist sensation to the skin, had a smooth external appearance, and exhibited excellent storage stability, with little change in external appearance even during storage. In contrast, the oil-in-water moisturizing milky lotions of Comparative Examples 17 and 18, in which either the component (A) or the component (B), essential in the oily composition according to the present invention, was not contained, did not realize satisfactory at least one of a rich full-body sensation immediately following application to the skin, a fresh feeling during use, absence of stickiness following use, a moist sensation following use, a smooth external appearance, and storage stability.

### [Industrial Applicability]

The oily composition and the cosmetic preparation according to the present invention exhibit a rich full-body sensation immediately following application to the skin, yield a fresh feeling during use, produce no stickiness following use, and impart a moist sensation to the skin.

Further, the oily composition and the cosmetic preparation according to the present invention have a smooth glossy external appearance, and undergo little change in external appearance even during storage.

## Claims

1. An oily composition comprising:
a component (A): an ester having a solid fat content (SFC) at 25°C within a range from 15 to 70% and having a value obtained by dividing a SFC at 35°C by the SFC at 25°C ([SFC (35°C)]/[SFC(25°C)]) that is within a range from 0.08 to 0.60;
a component (B): an oily gelling agent having a solidification point of 50°C or higher; and
a component (C): an oily component that is liquid at 25°C, wherein
an amount of the component (B) relative to 100 parts by mass of the component (A) is within a range from 0.05 to 10 parts by mass, and an amount of the component (C) relative to 100 parts by mass of the component (A) is within a range from 10 to 500 parts by mass.

2. The oily composition according to Claim 1, wherein the ester of the component (A) is a glycerol derivative.

3. The oily composition according to Claim 2, wherein the ester of the component (A) is glyceryl tri(caprylate/caprate/myristate/stearate).

4. The oily composition according to Claim 1, wherein a solidification point of the oily gelling agent of the component (B) is within a range from 60 to 90°C.

5. The oily composition according to Claim 1, wherein the oily gelling agent of the component (B) is a glycerol derivative.

6. The oily composition according to Claim 5, wherein the oily gelling agent of the component (B) is a material comprising eicosanedioic acid within a structure thereof.

7. The oily composition according to Claim 1, wherein the oily gelling agent of the component (B) is at least one selected from the group consisting of glyceryl (behenate/eicosanedioate), glyceryl tri(behenate/isostearate/eicosanedioate), polyglyceryl-10 (behenate/eicosanedioate), glyceryl tribehenate, 12-hydroxystearic acid, and candelilla wax.

8. A cosmetic preparation comprising the oily composition according to any one of Claims 1 to 7.

9. The cosmetic preparation according to Claim 8, wherein an amount of the oily composition in the cosmetic preparation is within a range from 1 to 40% by mass relative to a total mass of the cosmetic preparation.

10. A cosmetic preparation comprising:
a component (A): an ester having a solid fat content (SFC) at 25°C within a range from 15 to 70% and having a value obtained by dividing a SFC at 35°C by the SFC at 25°C ([SFC (35°C)]/[SFC(25°C)]) that is within a range from 0.08 to 0.60;
a component (B): an oily gelling agent having a solidification point of 50°C or higher; and
a component (C): an oily component that is liquid at 25°C, wherein
an amount of the component (B) relative to 100 parts by mass of the component (A) is within a range from 0.05 to 10 parts by mass, and an amount of the component (C) relative to 100 parts by mass of the component (A) is within a range from 10 to 500 parts by mass.

11. The cosmetic preparation according to Claim 10, wherein a total amount of the component (A), the component (B) and the component (C) in the cosmetic preparation is within a range from 1 to 40% by mass relative to a total mass of the cosmetic preparation.

12. The cosmetic preparation according to Claim 10, comprising an oily composition of any one of Claims 1 to 7.

13. The cosmetic preparation according to Claim 12, wherein an amount of the oily composition in the cosmetic preparation is within a range from 1 to 40% by mass relative to a total mass of the cosmetic preparation.

14. The cosmetic preparation according to Claim 10, further comprising a component (D): water.

15. The cosmetic preparation according to Claim 10, further comprising a component (E): a surfactant.

16. The cosmetic preparation according to Claim 10, further comprising a component (F): a powder.
